# EUROPEAN PATENT APPLICATION

(11) **EP 4 275 719 A2**
(43) Date of publication of application: **15.11.2023**
(21) Application number: 23195978.4
(22) Date of filing: 07.09.2023
(51) Int. Cl.: A61M 5/20

(54) **SAFETY MECHANISM FOR AN AUTOINJECTOR**

(71) Applicant: Ypsomed AG, 3401 Burgdorf (CH)
(72) Inventor: Scheurer, Simon, 3006 Bern (CH); Hostettler, Patrick, 3415 Hasle (CH); Klötzli, Urs, 3414 Oberburg (CH); Schüpbach, Simon, 3400 Burgdorf (CH); Zumstein, Dominik, 3014 Bern (CH); Allenspach, Marcel, 3400 Burgdorf (CH); Hulliger, Manuel, 4563 Gerlafingen (CH); Büchi, Florian, 3074 Muri bei Bern (CH); Etter, Leoni, 3011 Bern (CH); Lindegger, Stefan, 4950 Huttwil (CH)
(74) Representative: Kleiner, Stefan

(57) **Abstract**

The invention relates to a safety mechanism for an injection device. The safety mechanism comprising an assembly (2) defining a longitudinal axis and including a cap (10) releasably attachable to a distal end portion of the assembly (2). The cap (10) comprises a deflectable holding member (11) adapted to hold the cap (10) on the distal end portion. The assembly further comprises a rocker (38) tiltable around a center (35), the center dividing the rocker in a first portion (38.2) and a second portion (38.1). In a first tilt position of the rocker the cap (10) is hold on the distal end by the holding member (11) and in a second tilt position the holding member (11) is deflected by the second portion (38.1) such that the cap (10) is released from the distal end portion.

## Description

### FIELD OF THE INVENTION

The present invention relates to a safety mechanism for an injection device for injecting, delivering, administering, infusing or dispensing substances and/or liquids such as insulin, hormone preparations or vaccines. The safety mechanism comprising an assembly defining a longitudinal axis. The assembly includes a cap releasably attachable to a distal end portion of the assembly. The cap comprises a deflectable holding member adapted to hold the cap on the distal end portion.

### BACKGROUND OF THE INVENTION

A variety of diseases exist that require regular treatment by subcutaneous administration of a medicament, and several drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with syringes. Autoinjectors usually comprise a body for housing a syringe as well as a drive mechanism in order to move the plunger of the syringe upon actuation of the autoinjector. The drive mechanism typically comprises a source of drive, such as an electric motor or a mechanical strong spring for moving a transfer element, for example a rod, which acts on the plunger of the syringe. The prefilled syringe has a needle or cannula that is permanently attached to a first end of a syringe barrel or reservoir that is sealed at the opposing end by the plunger.

For safety and hygiene reasons it is desirable that the needle does not protrude from a housing of the autoinjector except for the time when the needle is used for injection of a medicament. Thus, either the autoinjector moves the needle out of the housing for the injection and back into the housing after injection or the autoinjector includes a needle guard which may be moved to unsheathe the needle for injection and which may be moved back to a needle covering position after the injection.

The majority of autoinjectors are configured as single use devices which incorporate both the syringe and the drive mechanism in the same housing. Such devices are usually disposable for hygiene reasons.

Disposable autoinjectors comprising an electric actuator or an electronic control require a source of energy which is usually in the form of a battery. However, in this case, the autoinjectors should not be disposed in the regular waste but have to be subjected to special disposal or to recycling, which is an additional burden to the patient. Further, disposing a battery, motor and/or electronics after a single use is a waste of resources and increases the costs of the therapy.

In order to account for a need to handle and dispose the autoinjector parts differently semi-reusable autoinjectors have been developed. Such autoinjectors typically comprise a reusable drive unit as well as a disposable syringe unit which may be releasably coupled or attached to the drive unit. The drive unit usually includes the drive mechanism and electronics whereas the syringe unit includes the syringe with the needle and a needle cover sleeve. The user can thus discard the syringe unit when it is empty or after use and can load the drive unit with a new syringe unit for a new upcoming injection.

WO 2010/046569 discloses a reusable autoinjector with a syringe unit to be coupled to a drive unit by a bayonet connection. Once a cam of the drive unit is inserted in a slot in the syringe unit housing a cover sleeve is unlocked and can be moved to unsheathe the needle for an injection. The syringe unit includes a unit-housing, a shield, a syringe holder and two springs biasing the shield to a covering position.

WO 2012/145685 discloses a reusable autoinjector with a disposable or single-use cassette and a reusable drive unit. The cassette includes an outer housing, an inner sleeve, a shield remover, a movable cover and a lock cap locking the syringe to the inner sleeve.

WO 2023/078720 A1 discloses an autoinjector comprising needle cover sleeve movable relative to an autoinjector housing and a cap removably attached to a distal end of the autoinjector. The needle cover sleeve is locked by a locking mechanism in a covering position in a shipping state to prevent undesired retraction of the needle cover sleeve and activation of the autoinjector dispensing mechanism. When the cap is pulled in a distal direction a gripping sleeve of the locking mechanism is moved to a release position thereby allowing an arm of a rocker to flex radially inwards which allows to pull off the cap and the gripping sleeve from the needle cover sleeve. This in turn unlocks the needle cover sleeve such that it can be pressed onto an injection site to start the dispensing.

### DESCRIPTION OF THE INVENTION

It is an objective of the invention to provide for a simple as well as a reliable releasing of a cap, which is initially mounted to a distal end of an injection device.

This objective is achieved by a safety mechanism, an injection device or a method according to the independent claims. Preferred embodiments are evident from the dependent claims.

The invention relates to a safety mechanism for an injection device, in particular for a semi-reusable autoinjector, for dispensing a liquid drug from a reservoir through a needle, the safety mechanism comprises an assembly defining a longitudinal axis. The assembly includes
- a cap releasably attachable to a distal end portion of the assembly and preferably of the injection device. The cap comprises a deflectable holding member adapted to hold or to fix the cap on the distal end portion;
- a rocker tiltable (pivotable) around a center and preferably arranged in a distal portion of the injection device. The center divides the rocker into a first and a second rocker portion.

The first portion includes an actuation surface to tilt the rocker when actuated and thus move the rocker around the center. The second portion can be engaged to the holding member, wherein in a first or initial tilt position of the rocker the cap is hold on the distal end by the holding member and in a second tilt position which is different from the first tilt position the holding member is deflected by the second portion such that the cap is released and thus can be removed from the distal end portion.

The safety mechanism featuring the rocker provides a reliable releasing or unlocking of the cap as the rocker can be in well-defined tilt positions. Namely, the rocker can be in a clearly defined and stable first, neutral or initial tilt position (e.g., in a shipping position) in which the second portion of the rocker does not deflect the holding member and thus the holding member can hold the cap onto the distal portion of the assembly. In the defined and stable second tilt position of the rocker the second rocker portion can engage and deflect the holding member such that the holding member no longer holds the cap in the initial cap position and thus the cap can be removed, either manually by a user force or by means of a biasing element.

By means of the rocker working as a seesaw one end portion of the rocker can be actuated, in particular pressed down or up or radially inwards or outwards and thus the other end portion of the rocker is moved in counter direction. The rocker can thus be used to redirect or to reroute a (automatic) release movement of a release member of the injection device or a manual release movement of the user.

This is further advantageous as the rocker can prolonge a movement of a moving member inside the injection device. This is in particular useful as the cap is usually located at a distal end or at an end portion of the injection device and thus remotely arranged from the dispensing or actuation mechanism of the injection device. Namely, a cap release movement can be conveyed distally from a drive to the cap without axial displacement (which would require an additional movable component) but exclusively by means of a pivoting axially fixed rocker.

The injection device may be either a manually actuated or an automatic injection device. Automatic devices or autoinjectors typically include automatic drive means such as an elastic element (for example a pre-tensioned spring) or an electric motor to drive a dispensing member to dispense the liquid drug form the syringe. Manual injection devices include a manual drive such as a dispensing button that has to be pressed by the user and by a human force to move the dispensing member in dispensing direction.

The injection device is preferably an autoinjector including a prefilled syringe. The prefilled syringe has a needle or cannula that is permanently attached to a first end of a syringe barrel or reservoir that is sealed at the opposing end by the plunger. The autoinjector is adapted to dispense the whole content of the syringe in a single injection stroke.

The safety mechanism comprises the assembly and may comprise further components such as a shield or locking mechanism, for example, to prevent unintentional access to an injection needle. The longitudinal axis of the assembly is preferably concentrically arranged to a longitudinal axis of the injection device. The assembly is preferably a subassembly of the injection device, for example a reservoir or syringe holding assembly, an assembly adapted to shield the injection needle or a reservoir unit or syringe unit releasably attachable to a drive unit. The assembly may further comprise components or elements of a drive unit comprising a drive. In this case the injection device is preferably a semi-reusable autoinjector (also named as semi-disposable autoinjector) having a reusable drive unit and a disposable syringe unit including the syringe with the drug.

In these semi-reusable injector concepts the syringe unit is typically not operational without a reusable drive unit of the injection device. To prepare the injection device for an injection the user has to attach or to couple the syringe unit to a drive unit. The syringe unit is usually attachable to the drive unit by means of a releasable lock such as a snap-fit connection, a thread or a bayonet fitting. The drive unit may include drive means adapted to dispense the liquid drug from the syringe hold inside the syringe unit when the syringe unit is attached to the drive unit.

The rocker working as a seesaw is tiltable supported or hold by the assembly of the injection device. The rocker may be bi-stable in either tilt position and may be supported or hold in place by a support structure of the assembly The rocker may be supported or hold in the center of the rocker by a flexible or resilient element. The first and second portion of the rocker are preferably arranged opposite to each other, for example, in form of protrusions, arms or projections. The rocker is preferably reversibly moveable. That means the rocker can be moved from the first tiled position to the second position and then back to the first position or to a third position. Alternatively, the rocker can only be tilted once from the first or shipping position to the second position and is then, for example, plastically deformed such that it cannot be moved back in the shipping position.

The deflectable holding member is adapted to hold the cap in place and to fix the cap on a distal end of the assembly and of the injection device. The deflectable holding member may be, for example, a clamp, arm, tongue or shell. Preferably, the holding member is elastically deformable such that it can be deflected to release the cap and then can deflect back to its original position.

The cap is released from the distal end portion if the holding member is in its second tilt position. The term "released" means the cap is free to be removed by the user from the distal end. Hence, the cap is not locked or otherwise hold. The term released does not mean that the cap is automatically moved or shifted off or pushed off by the release mechanism. Preferably, the cap remains on the distal end portion after release and has to be removed by the user.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

In the claims, the word "comprising" does not exclude other elements or steps, and the indefinite article "a" or "an" does not exclude a plurality. For example, "an arm" does not exclude the fact that there may be two arms that functionally or structurally fulfill the purpose of "an arm". The mere fact that certain elements or steps are recited in distinct claims shall not preclude the existence of further meaningful combinations of these elements or steps.

Preferably, the rocker is tiltable or pivotable around an axis perpendicular to the longitudinal axis. That allows to arrange the rocker in a space saving manner as in this orientation the rocker may extend along the longitudinal axis. Alternatively, the pivot axis may be parallel to the longitudinal axis. In this case the rocker is tiltable cross to the longitudinal device axis.

In a preferred embodiment the safety mechanism further comprises a reservoir holder adapted to fixedly hold the reservoir, preferably rotationally and axially fixed relative to the assembly and relative to an injection device housing. That means the reservoir (e. g. a syringe) does not move relative to housing during an injection process.

The rocker is preferably formed integral to the reservoir holder as a monolithic part. That means the rocker can be produced at the same time or in the same manufacturing step as the reservoir holder, in particular by plastic injection molding. That allows a simple and cost-saving manufacturing of the rocker.

Preferably, the assembly comprises a needle cover movable along the longitudinal axis relative to an injection device housing between a needle covering position, in which the needle is covered and a retracted position, in which the needle is exposed, wherein the needle cover is arranged outside and envelops the reservoir holder.

The needle cover may be implemented as shield or sleeve, or sleeve-shaped element adapted to cover or envelop a needle of a prefilled syringe or a carpule hold by the reservoir holder. The movement of the needle cover allows to switch between a safety state in which the needle is covered, and the needle does not protrude from a device housing and an injection state in which the needle is uncovered and exposes or protrudes from the housing and from the needle cover.

In a preferred embodiment the needle cover is the outermost part of the assembly and in particular of the reservoir holder. That means the assembly does not comprise any outer housing or shell but only the movable needle cover. This provides for a simple design.

The safety mechanism comprises preferably a deflectable actuation arm and a trigger member movable relative to the rocker and engageable to the actuation arm. Preferably, a relative axial movement between the trigger member and the actuation arm causes a radial movement of the actuation arm which in turn causes the rocker to move from the first tilt position to the second tilt position.

Furthermore, in a preferred embodiment a movement of the trigger member causes the actuation arm to press onto the actuation surface of the first portion of the rocker which preferably causes the rocker to tilt from the first tilt position to the second tilt position.

Hence, the rocker is actuated and moved to the second tiled position by the actuation arm, which in turn is moved by the trigger member. The trigger member may be automatically moved by a mechanism of the injection device such as, for example, an actuation process triggered by a successful medication check or a user confirmation. That means the injection device may include automatic drive means (e.g. electromagnetic actuator, an electric motor or a biased spring member) adapted to move the trigger member if a predefined condition is met or a threshold is exceeded. Alternatively, the trigger member may be moved manually by a user, for example, when the user presses a button.

The trigger member may be, for example, an elongated rod, a nut or a sleeve movable relative to an injection device housing. The trigger member can be coupled to the drive or to a user actuation member of the injection device. The actuation arm may be adapted to be movable in a predefined direction. That ensures that the rocker actuation surface is contacted in a well-defined manner. That in turn provides for a reliable actuation of the rocker, e. g. a movement of the rocker from the first to the second tilt position.

The trigger member is preferably movable along the longitudinal axis and the actuation arm is deflectable in a radial direction, preferably and essentially perpendicular to the longitudinal axis. As the trigger member is preferably connected to a mechanism of the injection device typically arranged in a proximal device portion the axial movement provides for an optimal and space-saving design.

In a preferred embodiment the trigger member comprises a first guiding surface (or first contact surface) and the actuation arm includes a second guiding surface (or second contact surface), wherein a movement of the trigger member along the longitudinal axis causes the first guiding surface to interact with the second guiding surface thereby deflecting the actuation arm in the radial direction to actuate directly or indirectly via intermediate member the rocker actuation surface. Hence, the first and second guiding surface can engage each other to allow a reliable deflection or movement of the actuation arm which in turn enables a reliable movement of the rocker by the deflected arm.

The guiding surfaces may be angled and in particular sloped. Hence the first and second guiding surface may be angled or sloped in the same slope angle or in the same direction such that a longitudinal movement of the trigger member causes the arm to deflect radially and thus essentially perpendicular to the longitudinal direction.

Due to the sloped guiding surfaces the longitudinal movement of the trigger member can be reliably transferred to a radial movement or to a movement in an angled direction with respect to the longitudinal axis. In concrete terms, the first sloped guiding surface can contact or engage the second sloped guiding surface and when the trigger member is further moved along the longitudinal axis the first sloped guiding surface is pressed onto the second sloped guiding surface thereby causing the two surfaces to slip on each other such that the longitudinal movement of the trigger member forces the actuation arm in a radial direction. Preferably, when the actuation arm is moved back its second sloped surface may be pressed onto the first sloped surface thereby moving the trigger member back along the longitudinal axis.

The first and the second sloped guiding contact surfaces may be positioned, for example, relative to the longitudinal axis in an angle of at least 20° and in particular in an angle between 40° and 50° to the longitudinal axis. Furthermore, the first guiding surface is preferably proximally faced, and the second guiding surface is distally faced.

In a preferred embodiment the safety mechanism comprises further a deflectable release arm adapted to engage directly the actuation surface of the rocker. That means the release arm is arranged inbetween the actuation arm and the rocker actuation surface and a deflection of the actuation arm causes a deflection of the release arm thereby moving the rocker from the first position to the second position. The release arm is preferably deflectable in a radial direction towards the rocker actuation surface.

Preferably, the release arm is formed in or is part of a first member of the assembly and the actuation arm is formed in or is part of a second member of the assembly. Hence, the actuation arm and the release arm belong to different assembly members and can be separated to each other. Preferably, the actuation arm is arranged on a housing part or drive mechanism part and the release arm is arranged on a reservoir holder or syringe unit.

The release arm preferably extends in a first direction from a connecting end to a free end and the first rocker portion extends preferably in a second direction (counter to the first direction) from the center to a free rocker portion. Such an arrangement allows a fail-safe implementation. If, for example, the injection device drops onto the ground an unintentional actuation of the rocker can be prevented as due to the different orientations of the rocker portion and the release arm one of the rocker or release arm may absorbed drop energy (impact energy) without tilting the rocker.

Moreover, as the release arm is radially arranged between the rocker actuation surface and the actuation arm and the release arm may act as radial extension to facilitate the access to the rocker actuation surface. Furthermore, the release arm is preferably elastically deflectable and due to a bias, the release arm may help to move back the actuation arm when the trigger member is moved back and away from the actuation arm.

In case the assembly includes a reservoir holder the release arm is preferably integrally formed with the reservoir holder as a monolithic part. The actuation arm and the reservoir holder are preferably made by plastic injection molding. That allows a simple manufacturing.

The assembly preferably comprises a (mechanical) sensor arranged in a distal portion of the assembly and adapted to sense the presence of the cap. The sensor is configured to provide a first sensor signal when the holding member holds the cap and a second signal different from the first signal when the cap is released from the holding member. The controller can thus determine whether the cap is mounted to the assembly.

The invention further relates to an injection device for dispensing the liquid drug from the reservoir. The injection device comprises, a plunger rod and a drive for driving the plunger rod and a safety mechanism as described above.

In a preferred embodiment the above descripted assembly is at least a part of a reservoir unit (or syringe unit) and a drive unit. The reservoir unit is adapted to hold the reservoir comprising the liquid drug. The injection device further comprises the drive unit comprising the drive and wherein the reservoir unit is releasably attachable to the drive unit.

The reservoir unit is preferably disposable, and the drive unit is preferably reusable. That means the injection device comprises two units allowing to establish a semi-reusable concept which allows to reduce waste. In contrast to conventional injection devices the semi-reusable device does not have to be discarded completely but only the reservoir unit can be disposed after use or if the drug is fully dispensed out of the reservoir. The drive unit can be reused for several injections.

The reservoir unit is releasably attachable to the drive unit. That means the two units can be detached and reattached without destroying elements. Non-limiting examples of releasable connections are a snap-fit, a bayonet connection or a thread.

In a preferred embodiment the reservoir unit includes a radially extending protrusion or radially extending rim portion and the drive unit includes a radially deflectable and flexible holding arm or clamping arm adapted to engage the protrusion or rim portion such that the reservoir unit is releasably hold by the drive unit via snap-fit connection.

The drive unit features preferably and additionally a lock to lock the snap-fit connection such that the syringe unit cannot be removed from the drive unit if the lock is activated. The lock may be implemented by a lock element adapted to engage a protrusion or recess of the reservoir unit.

Preferably, the reservoir inside the reservoir unit is a prefilled syringe including a syringe barrel with a permanently connected injection needle at the distal end thereof.

By preference, upon attachment the reservoir unit is held by the drive unit relative to a drive unit housing such that a movement of the reservoir unit in a dispensing direction is prevented. Furthermore, the reservoir inside the reservoir unit is fixedly hold such that the reservoir cannot move relative to the reservoir unit or relative to the drive unit during the injection process.

The drive unit preferably includes a support structure supporting or holding the drive and providing connection means to attach the reservoir unit to the drive unit. The drive unit preferably includes further an outer housing member, in particular two housing parts connectable to each other to form the outermost housing. The housing member is preferably devoid of any supporting capabilities and thus only forms an outermost shell for optical reasons or to protect the support structure from environmental influences. That means the drive unit is fully operatable with the support structure and without the housing member. The support structure may include a support or holding member for holding the housing member in place. However, such holding means do not provide a connection such that the housing supports any other components other than the two housing parts. That means all components of the drive unit are supported and hold in place by the support structure and not by the outermost housing members.

The two housing parts are preferably shell-like and connectable to each other, for example, by a snap-fit connection. Alternatively, the two members may be glued or welded to each other.

In a preferred embodiment the reservoir holder holds a syringe non-movable along the longitudinal axis. The syringe includes a syringe body or barrel which has at a distal end a narrow portion connecting the barrel to the injection needle. The reservoir holder includes a shoulder adapted to support or abut a distal end of the barrel in the region of the narrow portion. However, in a shipping or initial state a gap is present between the syringe barrel and the distal shoulder of the reservoir holder. The gap may be several millimeters, in particular 2 to 5 mm.

The injection needle is preferably shielded in a shipping state by a rigid needle shield (RNS) providing a sterile barrier which is to be removed prior use. The RNS is fixedly connected to a remover sleeve which in turn is connected to the cap. Hence, prior use the user removes the cap from the injection device and thus the remover sleeve together with the RNS are removed from the syringe.

If the user grabs the cap to remove the whole reservoir unit or to position the reservoir unit then the cap is typically moved a short distance of several millimeters too relative to the reservoir holder. This short movement is due to a play to account for manufacturing or assembly tolerances. During this cap movement a removal of the RNS and thus a removal of the sterile barrier or even a movement of the RNS relative to the injection needle is not (yet) desired.

As there is an initial gap between the syringe and the reservoir holder shoulder the RNS can travel a short distance together the whole syringe without impact to the sterile barrier. The mentioned gap between the syringe barrel and the shoulder is preferably larger than the distance the cap is movable relative to the reservoir holder (play for manufacturing or assembly tolerances).

In a preferred embodiment the drive unit includes a clamping arm that is adapted to engage a protrusion of the reservoir unit to hold the reservoir unit in an attached state. The clamping arm can be in an engaged position in which the clamping arm engages a protrusion of the reservoir unit to hold the reservoir unit. The clamping arm can be further in a released position in which the clamping arm does not engage the protrusion and thus does not hold the reservoir unit. Preferably, the clamping arm includes a sensor to sense when the clamping arm is in the engaged position and preferably the sensor is adapted to sense when the clamping arm is in the released position.

The sensor allows to detect a movement, a position or an engagement state of the clamping arm. Thus, it can be detected if the clamping arm was moved to engage the protrusion or if the protrusion is within a clamp of the clamping arm. That means it can be detected whether the reservoir unit is hold by the drive unit or not which is important for safety issues.

The arm may be, for example, shiftable, rotatable or pivotable between the release position and the engaged position. The drive unit may include two or more arms to reliably hold the reservoir unit in an attached state.

In a first preferred embodiment the sensor is a mechanical switch including an actuation switch which is actuated if the clamp of the clamping arm holds the protrusion and thus if the reservoir unit is attached to the drive unit. The mechanical switch may be actuated when the protrusion is inserted into a clamp of the clamping arm.

In a second embodiment the sensor is a strain gauge arranged on an outer surface of the clamping arm. The strain gauge allows to detect a deflection or a bending of the clamping arm during the attachment procedure when the protrusion of the reservoir unit is inserted into the clamp of the clamping arm. Thus, based on a strain gauge signal an engagement movement and thus the engagement of the clamping arm with the protrusion can be detected.

In a third embodiment the sensor is implemented by a light guide or fiber optic. In this case a light guide is interrupted by the clamping arm when it is moved during the attachment process. Thus, it can be detected when the clamping arm is moved by the protrusion before the clamp engages the protrusion and thus it is detected when the reservoir unit is attached to the drive unit.

The drive may be a manual drive with an elastic deformable drive element, which biased manually by a user prior to an injection. The drive element may be, for example, a spring or an elastic deformable elastomer element. The spring may be, for example, a biased mechanical spring such as a spiral spring, compression spring or tension spring.

Alternatively, the drive is an automatic drive, for example, including an electric motor or electromagnetic coil actuator. The automatic drive may include further a controller adapted to control the motor or actuator.

In a preferred embodiment the automatic drive is adapted to rotate a drive member which is operatively coupled (direct or indirect via intermediate member) to the plunger rod and the trigger member, wherein rotating the drive member in a first rotational direction causes the plunger rod and the trigger member to be moved in the first direction along the longitudinal axis and rotating the drive member in a second, counter rotational direction causes the plunger rod and the trigger member to be moved in the second direction along the longitudinal axis.

Hence, with one single automatic drive the plunger rod can be moved back and forth and preferably the trigger member can be moved back and forth at the same time. That allows to lock and unlock the cap on the cover sleeve (via intermediate member, if any) and with the same automatic drive the plunger rod can be driven to dispense the drug form the reservoir. That means the automatic drive can be used to rotate the drive member to move the trigger member, preferably before an injection to lock or unlock the cap and subsequently the automatic drive can be used to dispense the drug during an injection or for preparation steps.

Preferably, the drive member is in treaded engagement with the plunger rod, such that the plunger rod is exclusively shifted along the longitudinal direction without rotation when the drive member is rotated. The trigger member is preferably directly or indirectly coupled to the plunger rod such that a longitudinal movement of the plunger rod in the first direction causes to move the trigger member in a first longitudinal direction and movement of the plunger rod in the second longitudinal direction causes to move the trigger member in a second longitudinal direction.

The invention further refers to a method for operating a safety mechanism for an injection device for dispensing a liquid drug from a reservoir through a needle as described above. The method includes the steps of
a. Providing an injection device defining a longitudinal axis;
b. Moving a trigger member of the injection device along the longitudinal axis
c. Tilting, by the trigger member movement, a first tilt position to a second tilt position
d. Releasing, by the tilting movement of the rocker, a cap releasably attached to a distal end portion of the injection device by deflecting a holding member initially holding the cap on a distal end of the injection device.

Preferably, step c) includes a deflecting, by the trigger member movement, an actuation arm and thereby moving a rocker from a first tilt position to a second tilt position.

The present disclosure includes a use of a reservoir unit with a drive unit comprising a drive for driving the plunger rod and wherein the reservoir unit and the drive unit comprise the above described safety mechanism and wherein the reservoir unit is releasably attachable to the drive unit. The drive unit preferably includes a movable clamping arm that can be in an engaged position in which the arm engages a protrusion of the reservoir unit to hold the reservoir unit in an attached state and wherein the clamping arm can be in a released position in which the protrusion is released and wherein the drive unit includes a sensor configured to sense when the clamping arm is in the engaged position.

The use of a reservoir unit with the drive unit comprises the drive provided by an electric motor and a controller adapted to control the motor.

In a further preferred embodiment of the use of a reservoir unit with the drive unit the motor is preferably adapted to rotate a drive member which is operatively coupled to the plunger rod and the trigger member, wherein rotating the drive member in a first rotational direction causes the plunger rod and the trigger member to move in a first direction along the longitudinal axis and rotating the drive member in a second, counter rotational direction causes the plunger rod and the trigger member to move in a second direction along the longitudinal axis.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a perspective view of an autoinjector according to the invention;
- Fig. 2: depicts a perspective view of a syringe unit of the autoinjector;
- Fig. 3: depicts an exploded view of the syringe unit;
- Fig. 4: depicts a second embodiment of the syringe unit;
- Fig. 5: depicts a sectional view of a drive unit of the autoinjector;
- Fig. 6a: depicts a sectional view of the autoinjector with the syringe unit attached to the drive unit;
- Fig. 6b: depicts a sectional view of the autoinjector of figure 6a wherein the plane for the sectional view is offset and parallel a plane in the center of the longitudinal axis;
- Fig. 6c: depicts a sectional view of the autoinjector of figure 6a wherein the plane is offset and parallel the plane in the center of the longitudinal axis;
- Fig. 7: depicts the autoinjector with a syringe unit locked to the drive unit and without cap and a released cover sleeve before an injection;
- Fig. 8: depicts the autoinjector after a dispensing of a dose;
- Fig. 9a: depicts the drive unit without the outer shell parts and
- Fig. 9b, 9c: depict the upper and lower shell part of the drive unit.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

In the present description the term "distal" refers to the side where the injection needle is located. This is on the left-hand side in the figures. The term "proximal" refers to the opposite side or rear end of the device and is on the right-hand side in the figures.

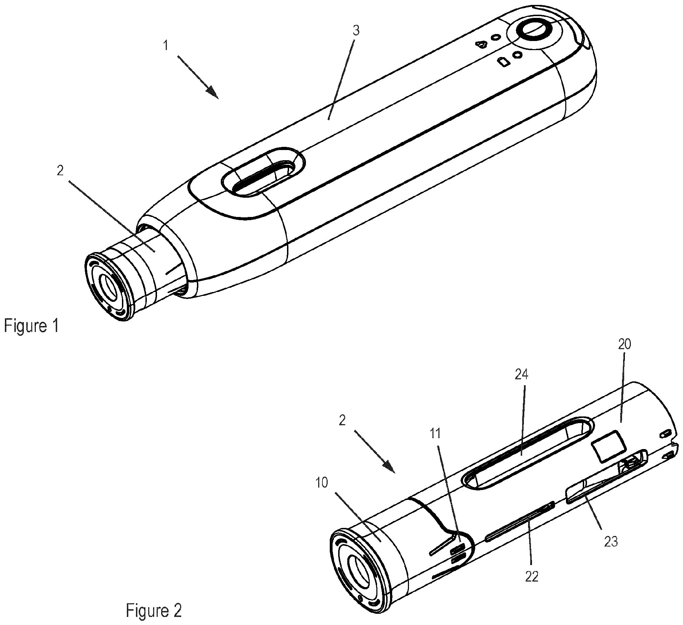

Figure 1 shows a perspective view of an injection device of a first embodiment of the invention in form of a semi-reusable autoinjector 1. The autoinjector 1 comprises a disposable syringe unit 2 and a reusable drive unit 3. As shown in figure 1 the drive unit 3 has an elongated housing defining a longitudinal axis and comprises a button 4 at its proximal end. The drive unit further includes an opening or cavity adapted to accommodate a proximal portion of the syringe unit 2 to attach or couple the syringe unit 2 to the drive unit 3.

In the following the structural features of the syringe unit 2 and the drive unit 3 will be descripted in detail. Subsequently, the function of the semi-reusable autoinjector 1 will be explained.

### Syringe Unit

Figure 2 depicts a perspective view of the syringe unit 2 without the drive unit. The syringe unit 2 has an elliptical form in the cross section and extends along a longitudinal axis. On a distal end the cap 10 is mounted in an initial state or shipping state. The proximal end includes a holding structure as described below to releasably attach the syringe unit 2 to the drive unit 3.

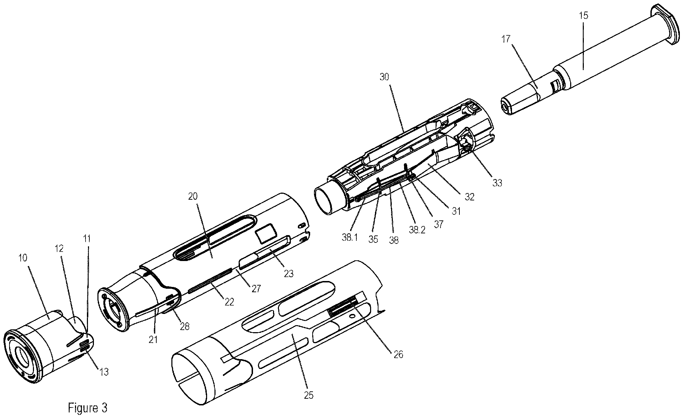

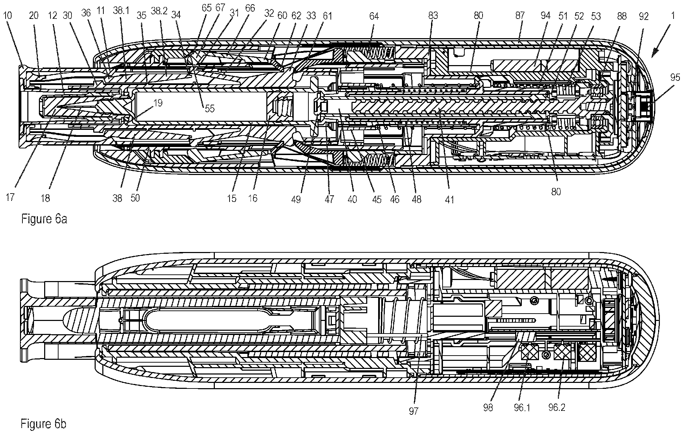

Figure 3 depicts an exploded view of the syringe unit 2. As shown in figure 3 the syringe unit 2 includes a syringe holder 30 (reservoir holder), a cover sleeve 20 coaxially arranged around the syringe holder 30 and movable relative thereto along the longitudinal axis, a label 25 wrapped around the cover sleeve 20, the cap 10 with a RNS remover and a prefilled syringe 15 (figure 6a) comprising a liquid drug and a rigid needle shield 17 (RNS) initially mounted on the injection needle. The syringe holder 30, the cover sleeve 20 and the cap 10 are individually injection-molded plastic parts which are all made of the same material, preferably polypropylene.

The prefilled syringe 15 with a barrel made of glass is rotationally and axially fixed inside the sleeve-shaped syringe holder 30. For that purpose, the syringe holder 30 includes clamping elements (not shown) pressing on an outer surface of the syringe barrel. The syringe holder 30 provides a bearing surface 19 (see figure 6a) on its inside adapted to abut a shoulder of the syringe barrel. The syringe holder 30 further features longitudinal guiding rails (not shown) on its outside for the cover sleeve 20 such that the cover sleeve 20 can be shifted exclusively in the longitudinal direction relative to the syringe holder 30.

As shown in figure 3 and 6a the syringe holder 30 includes two oppositely arranged and in longitudinal direction extending rockers 38 or seesaws. Each rocker 38 is pivotable around a center 35 which is formed by a flexible or elastic connection fixing the rocker 38 on an outer surface of the syringe holder 30. The center 35 divides the rocker 38 into a distal portion 38.1 or distal arm and a proximal portion 38.2 or proximal arm. The distal portion 38.1 is adapted to engage and deflect a cap holding tongue 11. For this purpose, the distal portion 38.1 includes a nose 36 (see figure 6a) adapted to engage a projection of the cap holding tongue 11 to disengage a snap-fit connection between tongue 11 and sleeve 20 or holder 30.

As best shown in figure 6a the proximal portion of the rocker 38 includes an actuation surface 34 adapted to be contacted by a free end of a release arm 32. The rocker 38 is adapted to be tilted or pivoted around the center 35 between a first or initial tilt position where the distal portion 38.1 is in a radially inward position and a second tilt position where the distal portion 38.1 is in a radially outward position.

The two radially deflectable release arms 32 (see figure 3 or 6a) are arranged on opposite sides of the syringe holder 30 and include each a distally directed free end with an end bulge 31 and a connecting end which connects the release arms 32 to the syringe holder 30. At the free end and lateral to the bulge 31 are two side support surfaces 37 on both sides of the bulge 31 to guide the release arms 32 as descripted below. The rocker 38 and the release arm 32 are integrally formed in the syringe holder 30 as a monolithic part. That means the syringe holder 30 with the rockers and the release arms is injection molded as one single part as best shown in figure 3.

In an initial state the release arms 32 are in a non-deflected state and in a locking position and prevent a movement of the cover sleeve 20 relative to the syringe holder 30 in a proximal direction. Hence, the cover sleeve 20 shields or covers the injection needle of the syringe 15 in a covering position. For that purpose, a free end of the locking arms 32 abut stop surfaces (not shown) inside the cover sleeve 20. As will be descripted below once the locking arms 32 are deflected radially inwards the cover sleeve 20 can be pushed proximally.

The syringe holder 30 further includes two oppositely arranged and radially protruding end ledges 33 at a proximal end of the syringe holder 30 for engagement with clamping arms 61 (figure 6a) inside the drive unit 3.

As shown in figure 3 the cover sleeve 20 has a non-circular cross section and in particular an elliptical form in a plane perpendicular to the longitudinal axis. The cover sleeve comprises a medicament window 24 allowing the user to view the fill level of the syringe inside the syringe unit. The cover sleeve further includes a first lateral opening 21 for nose 36 of the rocker 38 to reach the cap holding tongues 11, a second opening 22 for access to the release arms 32 and a third opening 23 for access to the end ledges 33 from outside. The label 25 is wrapped around an outer surface of the syringe unit. The label includes a RFID code 26 integrated in the label and positioned in a proximal portion of the label 25. The code 26 includes information about the syringe unit for identification and may include further information about the drug inside the syringe, the volume, expire date and dispensing parameter such as injection speed and holding time The code 26 may have further country-specific information such as a user language to adjust a user interface language.

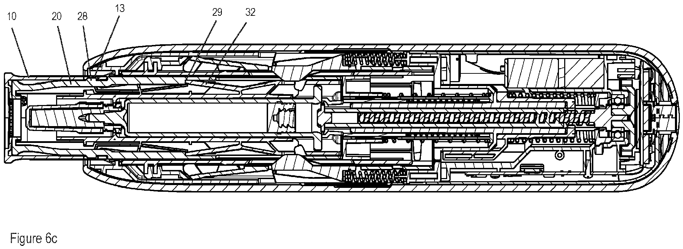

At a distal end of the cover sleeve 20 the cap 10 is releasably attached by a snap-fit connection. The cap 10 includes on two sides two deflectable holding members in form of holding tongues 11 on each side. The tongues 11 include on the free end an indentation or recess 13 adapted to be snapped to a protrusion 28 arranged on an outer surface of the cover sleeve 20 (also shown in figure 6c). Each tongue further includes a lobe projecting in the first opening 21 in the cover sleeve if the cap is mounted. The release of the snap-fit will be descripted below with respect to the decapping of the syringe unit 2. The cap 10 includes in its inside a sleeve-shaped holder 12 (RNS remover) connectable to the rigid needle shield (RNS) 17 of the syringe 15 such that the RNS 17 is removed with the cap 10 if the cap is released from the cover sleeve 20. The holder 12 is made of an elastomer and is shrunken onto the RNS during assembly of the syringe unit. As an outer cap housing is made of plastics the holder 12 and the outer cap housing from two-component injection molded part. The cap 10 with the holder are descripted in the patent application EP23165348.6 (Ypsomed) in detail which is incorporated herein by reference.

As an alternative the holder can be made of metal and coaxially arranged to the outer cap housing as the holder 12 descripted above. In this case the metal holder engages the RNS 17 and the RNS is removed together with the cap 10 if the cap is released from the needle cover.

The syringe 15 includes the syringe barrel and inside the barrel a movable piston 16 (figure 6a). The injection needle 18 (figure 6a) is non-releasably connected to a distal end of the syringe barrel. In an unused state the injection needle 18 is covered by the RNS 17, see figure 6a. The latter in turn is axially and radially fixedly connected to the cap 10 by the holder 12 as described above once the syringe 15 is mounted to the syringe holder 30.

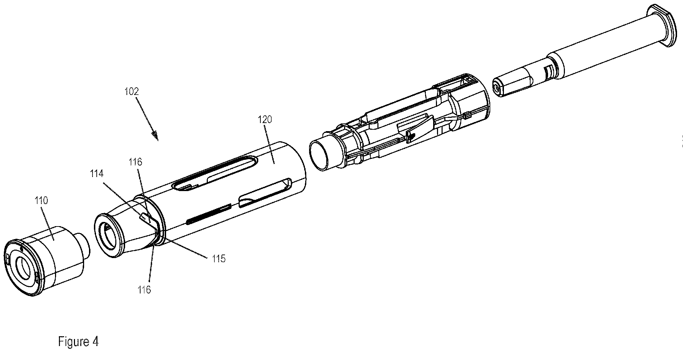

Figure 4 depicts an alternative syringe unit 102. In contrast to the syringe unit described with respect to figures 2 and 3 the syringe unit 102 shown in figure 4 includes a cap 110 mounted to the cover sleeve 120 by a bayonet-like connection.

Instead of the tongues the cap 110 includes two oppositely arranged and radially inward protruding cams or projections (not shown) adapted to project into corresponding grooves 114 in a distal cover sleeve portion.

The grooves 114 are U-shaped. Each groove 114 include a straight groove portion 115 which is perpendicular to the longitudinal axis and on both ends of the straight groove end groove portion 116 are arranged which are angled to the longitudinal axis.

The 110 cap can be mounted and released from the cover sleeve 120, respectively, by a combination of an axial and rotational movement. Hence, in contrast to the embodiment above with the rocker the cap 110 shown in figure 4 can be removed from the cover sleeve manually by a user force any time without a release mechanism with a rocker as described above.

If the user grabs the cap 110 and rotates the cap the cam enters the angled groove portions 116 and the cap 110 additionally moves axially in distal direction away from the cover sleeve end due to the angled groove portions 116. The user can then completely remove the cap 110 from the cover sleeve 120. All other features and functions of the syringe unit are the same as descripted with respect to the first embodiment shown in figure 3.

### Drive Unit

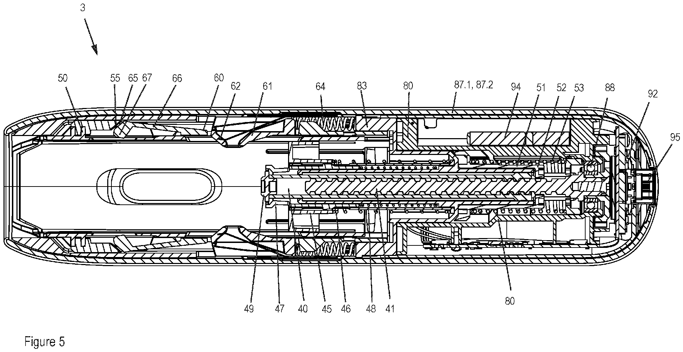

Figure 5 depicts a sectional view of the drive unit 3 without syringe unit. The cut of the sectional view runs along the longitudinal axis of the drive unit. The drive unit 3 comprises a support structure 80 including an outer housing cover or shell 87.1, 87.2 and inside the cover a mechanics sleeve 83 is fixedly connected to the support structure 80. Furthermore, the drive unit 3 includes the opening on its distal side adapted to accommodate a proximal portion of the syringe unit 2.

The support structure 80 guides a gripper sleeve 60 is immovably arranged. Coaxially and inside the gripper sleeve 60 a trigger sleeve 50 is arranged which is movable relative to the supporting structure 80 along the longitudinal axis. Again, inside the trigger sleeve 50 a movable cover sleeve connector 45 is located and biased in distal direction by a cover sleeve spring 46. The spring abuts on its distal end the cover sleeve connector 45 and on its proximal end a radial wall of the mechanics sleeve 83. A movable sleeve-shaped syringe connector 47 is coaxially arranged inside the cover sleeve connector 45 and biased distally by a syringe connector spring 48 coaxially inside the cover sleeve spring 46 and proximally supported by the mechanics sleeve 83. The spring 46 ensures that the syringe connector 45 pushes the syringe 15 of an inserted syringe unit 2 in distal direction.

In a proximal portion the support structure 80 supports and guides a sleeve-shaped trigger sleeve connector 51 movable along the longitudinal axis, a drive assembly with an electric motor (not shown) and a gear (not shown) connecting the motor with a threaded rod 41, a battery 94, the treaded rod 41 and a plunger rod 40 threadedly connected with the threaded rod 41. The support structure 80 further accommodates an electronic module 92 with a controller configured to control the electronic motor and providing information to the user via a display or LED 95 or a communication module (not shown). The trigger sleeve connector 51 is fixedly and immovably connected to the trigger sleeve 50 and distally pressed onto a proximal end of the plunger rod 40 by a trigger sleeve spring 53. The plunger rod 40 is non-rotatably guided by the mechanics sleeve 83 and comprises on its distal end a flange 49 adapted to engage the piston 16 (see figure 6a) during dispensing.

As shown in figure 6b inside the proximal portion of the support structure 80 a detection pin 97 is axially movable by the cover sleeve connector 45. The detection pin 97 includes a cam 98, which can interact with a position sensor implemented by two mechanical switches 96.1, 96.2 (figure 6b) arranged along the longitudinal axis. The presence of a syringe unit 2 inside the drive unit 3 is thus detectable via detection pin 97 and the first switch 96.1 which provides a corresponding signal to the controller of the electronic module. Furthermore, a retraction of the cover sleeve 20 can be detected by the second switch 96.2 as the cover sleeve 20 retraction further moves the pin 97 with its cam 98 in proximal direction.

Additionally, in a distal end portion a mechanical switch (not shown) held by the support structure 80 to sense the presence or absence of the cap 10 of an attached syringe unit 2.

The gripper sleeve 60 includes two axially extending and deflectable clamping arms 61 having a clamp 62 at their distal free end adapted to accommodate the syringe holder ledges 33. The clamping arms 61 can be deflected radially outwards allowing the clamps 62 to snap onto the ledges 33 or to release the ledges 33. Furthermore, each clamping arm 61 provides a support for an axially aligned gripper sleeve spring 64 which biases the clamping arms 61 in a holding position.

Each clamping arm 61 includes a mechanical sensor with an actuation rod to detect when the syringe holder ledges 33 are inserted in the clamps 62. Alternatively, a strain gauge or a piezoelectric strain gauge may be arranged on a radial outer surface of the clamping arm 61 and electrically connected to the controller in the electronic module. Based on a strain gauge signal or piezoelectric sensor signal a deflection of the clamping arms 61 can be detected. This allows to determine if the clamping arms 61 were pivoted by the ledges 33 during insertion of the syringe unit 2 into the drive unit 3. That in turn allows to conclude if a syringe unit is completely inserted into and attached to the drive unit.

As a further alternative light guides (fiber optics) may be used to detect the clamping arm deflection. That means a light guide is arranged from the PCB to each clamping arm 61 such that a deflection of the clamping arms 61 interrupts the light guide. The corresponding signal allows to determine that the clamping arms were deflected by the ledge 33 of the inserted syringe unit 2.

The gripper sleeve 60 further includes two oppositely arranged and radially inward deflectable actuation arms 66. The actuation arms 66 extend essentially in the longitudinal axis and include on their distal free end 67 a guiding surface 65 or sloped contact surface. The actuation arms 66 are deflectable radially inwards upon contact with a counter guiding surface 55 or counter sloped contact surface of the trigger sleeve 50. The free ends 67 of the actuation arms 66 further comprise each a radially inward directed contact surface to abut the end bulge 31 of the release arm 32 of the syringe unit 2 as described further below.

The trigger sleeve 50 is axially guided by support structure 80 and shiftable relative thereto. For this purpose, the trigger sleeve 50 includes longitudinal guiding rails (not shown) engaging longitudinal grooves (not shown) in support structure. As it can be seen in figure 5 and 6a the trigger sleeve 50 incudes oppositely arranged contact elements featuring the counter guiding surface 55 or counter sloped contact surface. As mentioned above the trigger sleeve 50 is further immovably connected to the trigger sleeve connector 51. As the trigger sleeve connector 51 is biased by the trigger sleeve spring 53 the trigger sleeve 50 is biased too in distal direction. The trigger sleeve connector 51 in turn is connected to the plunger rod via ledges 52 which engage a proximal shoulder of the plunger rod 40 such that an axial movement of the plunger rod 40 in proximal direction also moves the trigger sleeve 50 proximally.

In the state shown in figure 5 the drive unit 3 is ready to be loaded with a syringe unit 2. The controller displays the current state to the user by means of a steady green light.

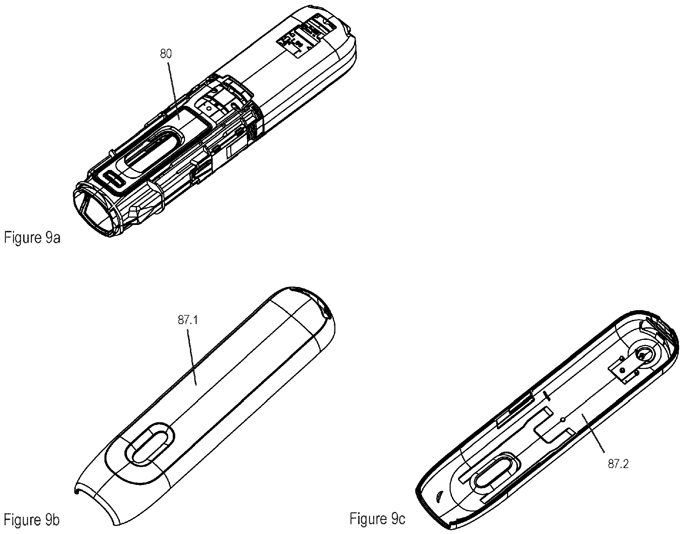

The support structure 80 supports and holds the whole drive mechanism including the above-mentioned trigger sleeve 50 and gripper sleeve 60 as well as the motor and the electronic module in a proximal portion. The drive unit 3 further includes an outermost shell comprising two shell parts 87.1, 87.2 that are connected to each other by a snap-fit connection so that the parts are tightly fixed to each other. The support structure with the drive mechanism is depicted in figure 9a whereas the upper shell part 87.1 is shown in figure 9b and the lower shell part 87.2 in figure 9c.

The shell parts do not support any internal component but are fixedly connected to the support structure 80 and act as a grip for the user. The shell parts 87.1, 87.2 may be adjusted in color, in the form, size and appearance for a specific use case.

### Function

In order to prepare the autoinjector 1 for an injection the user attaches a syringe unit 2 to the drive unit 3. The electronics in the drive unit 3 are switched from an inactive state or sleep mode to an active mode upon detection of the syringe unit by the switch 96.1 (figure 6b) of the position sensor. Alternatively, the user can press the button 4 to activate the electronics.

Figure 6a, 6b and 6c depict sectional views of the drive unit 3 with an inserted syringe unit 2. In figure 6a the cut for the sectional view runs along the longitudinal axis in a center plane. The plane of the section for Figure 6b and 6c is parallel but in a radial distance to a center longitudinal axis and hence offset from and parallel to the center plane.

In the state shown in figures 6a, 6b and 6c the syringe unit 2 has been inserted with its proximal end portion into the distal opening of the drive unit 3. In the state shown in figures 6a, 6b and 6c the proximal ledges 33 of the syringe holder 30 are snapped into the clamps 62 of the gripper sleeve clamping arms 61. The syringe unit 2 is thus held in the drive unit 3 by the clamping arms 61. However, in the state in figures 6a, 6b and 6c the syringe unit 2 can still be pulled out of the drive unit 3 by overcoming a holding force. Hence, if the user pulls the syringe unit 2 in a distal direction the clamping arms 61 are deflected radially. The ledges 33 of the syringe holder 30 are therefore released from the clamps 62 and the syringe unit 2 can be removed from the drive unit 3.

The presence of the ledges 33 in the clamps is detected by the controller as the ledges abut a cam of the mechanical switch inside the clamps 62. The controller thus receives a signal that a syringe unit is correctly and completely inserted into the drive unit. Alternatively, if the detection is provided by a strain gauge on the clamping arms 61 or a light guide interrupted by the deflecting clamping arms 61 an engagement movement of the clamping arms is detected and hence the controller can determine that a syringe unit 2 is attached based on the strain gauge or light guide signal.

That means in this state the syringe unit 2 is held inside the drive unit 3 but not yet locked inside the drive unit 3. The mechanical switch in the distal end portion of the support structure 80 is actuated by the cap 10 of the inserted syringe unit. Hence, the controller can determine that a cap 10 is mounted on the inserted syringe unit 2.

In the initial state or shipping state the cover sleeve 20 is locked into the covering position. This is shown in figure 6c where the plane for the sectional view is offset a center axis. As it can be seen in figure 6a a distally oriented end of the release arm 32 abut or engage a proximally oriented stop surface 29 of the cover sleeve 20. A force in proximal direction acting on the cover sleeve 20 is thus absorbed by the release arm 32 and hence the cover sleeve cannot be moved out of the covering position towards the retracted position.

Furthermore, in an initial state or shipping state of the syringe unit the syringe barrel does not abut the bearing surface 19 and hence between a distal end of the syringe barrel and the bearing surface 19 of the syringe holder 30 is a gap. The gap may be several millimeters, in particular 2 to 5 mm.

The reason for the presence of the gap is that the device cap 10 has to be movable a short distance if the user grabs the cap 10 to remove the (unused) syringe unit or to position the syringe unit. The travel of the cap is due to a play provided between the parts to account for manufacturing or assembly tolerances. During such a cap movement a removal of the RNS 17 and thus a removal of the sterile barrier is not desired.

As there is an initial gap between the syringe barrel and the bearing surface 19 the RNS (with the needle) can travel a short distance without impact to the sterile barrier. That means the gap between the syringe barrel and the syringe holder bearing surface 19 is preferably larger than a maximal play of the cap (cap travel relative to the syringe holder).

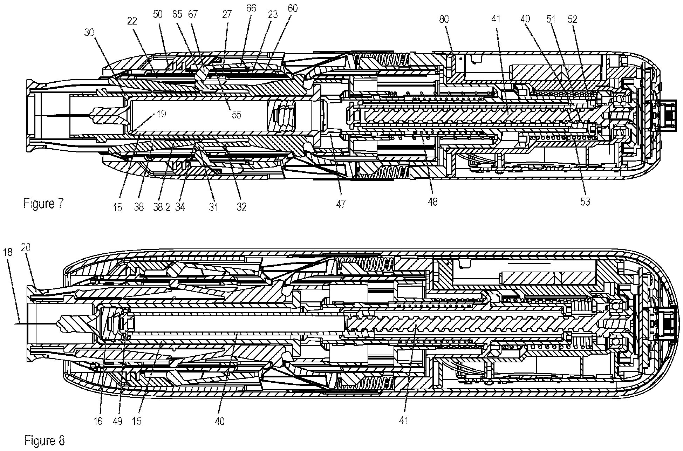

Figure 7 depicts the autoinjector 1 in a locked state without cap. When the syringe unit 2 is inserted as described above the proximal end of the syringe unit 2 moves the detection pin 97 proximally and thus the switch 96.1 of the position sensor automatically sends a signal to the controller in the electronic module which detects the presence of the syringe unit 2 inside the drive unit 3. The controller wakes up the electronics and sets the device in an active state. A code reader (not shown) in form of an NFC reader inside the drive unit 3 reads the RFID code 26 of the syringe unit 2. Optionally, a temperature sensor is additionally integrated in the syringe unit RFID code configured to sense the current syringe unit temperature. Subsequently, the controller of the drive unit sends the read medication information and eventually the temperature information from the read code 26 to an external device to verify the drug or medication (and eventually the temperature information) with predefined values or tables. Alternatively, the controller compares the read information from the code 26 with predefined information stored in a memory inside the drive unit 2.

In case of successful medication and eventually temperature verification the controller receives an enable signal (either from the external device or from the internal comparison). The controller then controls the electric motor to rotate the threaded rod 41 to move the trigger sleeve 50 a short distance of several millimeters in proximal direction. Such a trigger sleeve 50 movement is achieved by a kinematic chain as described as follows.

The motor rotates a pinion and the gear transfers the rotation to the threaded rod 41 which in turn moves the non-rotating plunger rod 40 in proximal direction, preferably an axial travel of about 5 mm. As the trigger sleeve connector 51 is connected via its ledges 52 to a proximal shoulder of the plunger rod 40 the latter causes the trigger sleeve connector 51 to move and thus the trigger sleeve 50 is shifted in proximal direction too. Furthermore, the trigger sleeve spring 53 is compressed during the proximal movement.

The proximal movement of the trigger sleeve 50 brings the counter sloped guiding surfaces 55 of the trigger sleeve 50 into contact with the sloped guiding surfaces 65 of the actuation arms. As the two sloped surfaces 65, 55 slide along each other the actuation arms 66 are forced to deflect radially inwards and the actuation arms free ends 67 are pressed radially under the trigger sleeve part with the guiding surface 65 as shown in figure 7 when the trigger sleeve 50 is further moved in proximal direction. The radial inward directed contact surface of the free ends 67 of the deflected actuation arms in turn encounter the end bulges 31 of the release arms 32 thereby deflect the release arms 32 radially inwards too. Subsequently, the inwardly moving release arms 32 abut the rocker actuation surface 34 and tilt the rocker 38 from the first tilt position towards the second tilt position such that the proximal portion 38.2 of the rocker with the actuation surface 34 is pivoted radially inwards.

Consequently, the distal portion 38.1 of the rocker moves radially outwards and hence deflects the holding tongues 11 of the cap 10 radially outwards. That in turn means that the snap-fit connection holding the cap 10 onto the distal end of the cover sleeve 20 is released as the recesses 13 of deflected holding tongues 11 no longer engage the protrusions 28 on the cover sleeve 20. The user can thus pull off the cap 10 form the autoinjector.

When the cap 10 is removed the cam of the mechanic switch is no longer actuated and hence the controller derives from the corresponding switch signal that the cap 10 is removed. Alternatively, if the sensor is implemented with a light guide the removal of the cap 10 interrupts the light guide or alternatively allows an uninterrupted light guide and hence a corresponding signal indicates that the cap is no longer present. Additionally, in case a light guide is used the cap can be illuminated by the light guide if the cap is made of a translucent or transparent plastic. That means the controller may activate a light flashing or constantly illuminating the cap in a colour signaling the user that the cap is released and can be removed. If the cap is removed the light guide is interrupted and a cap removal signal is generated for the controller.

If the cap 10 is mounted the syringe 15 inside the syringe holder 30 is hold in place by the cap 10 via holder 12 and RNS 17 and thus the distally biased syringe connector 47 cannot move the syringe 15 relative to the syringe holder 30. However, when the cap 10 is removed the syringe 15 is no longer hold and due to the syringe connector spring 48 the syringe connector 47 moves the syringe 15 relative to the syringe holder 30 distally and thus the gap between the distal end of the syringe barrel and the bearing surface 19 disappears. That means upon cap removal the syringe connector urges the syringe 15 against the bearing surface 19 such that the syringe 15 is fixedly hold between the biased syringe connector 47 and the bearing surface 19.

The radially inwardly deflected release arms 32 have further the effect that they no longer engage the stop surface 29 (figure 6c) of the cover sleeve 20 and thus allow for the cover sleeve 20 to move out of a covering position and move inside the support structure 80 in proximal direction relative to the syringe holder 30.

At the same time the radially inwards deflected actuation arms 66, namely a proximally oriented surface of the free ends 67 of the actuation arms 66 abut a rib 27 of the cover sleeve 20. The rib 27 is located between the opening 22 and opening 23 in the cover sleeve shown in figure 3 and in the sectional view in figure 6c. That means in this locked state the syringe unit 2 cannot not be pulled out as the free end 67 abuts the rib 27 of the cover sleeve and hence the syringe unit 2 is locked in the drive unit.

The cap 10 of the autoinjector 1 is now removed and the autoinjector is ready for an injection. The controller displays a corresponding notification on the display the LED 95 constantly lights.

As a next step the user places the distal end of the cover sleeve 20 onto an injection site and pushes the autoinjector 1 towards the injection site. This causes the cover sleeve 20 to move proximally from the distal covering position into support structure 80 (push on skin) and compresses the cover sleeve spring 46 which biases the cover sleeve 20 in distal direction via cover sleeve connector 45. The retraction of the cover sleeve 20 is possible as the end ledges 31 of the release arms 32 are hold radially inwards by the deflected actuation arms 66 (figure 7). When the cover sleeve 20 is in its retracted position the injection needle 18 protrudes from the cover sleeve as shown in figure 8.

Upon push on skin the cover sleeve connector 45 is moved proximally and thus the detection pin 97 is further moved proximally. The further movement brings the cam 98 in contact with the second switch 96.2 (figure 6b) which triggers the controller in the electronic module to start the injection. That means the controller drives the electric motor in dispensing direction and thus rotates the threaded rod 41 and thereby moves the plunger rod 40 in distal direction.

The controller drives the plunger rod distally with a low torque. Upon contact of the plunger rod flange 49 with the piston 16 inside the syringe barrel the motor current increases due to the motor controlling. That is a signal for the controller that the plunger rod 40 is correctly positioned. Subsequently, the motor drives the plunger rod to dispense the predefined dose.

During dispensing the distal flange 49 of the plunger rod 40 moves the piston 16 inside the syringe 15 in distal direction and thus dispenses liquid drug through the injection needle out of the syringe 15. If the piston 16 abuts a distal end inside the syringe barrel the current of the motor controlling increases and the controller stops to drive the plunger rod distally. This end of injection condition is shown in figure 8. As it can be seen the plunger rod 40 and thus the piston 16 inside the syringe barrel is in a distal end position.

The distal movement of the plunger rod 40 moves the trigger sleeve 50 via trigger sleeve connector 51 in distal direction to its initial position. That means the actuation arms 66 can move radially outwards back into their non-deflected position. However, as the cover sleeve 20 is in its retracted proximal position the release arms 32 are prevented from being moved back into the initial non-deflected position by the side support surfaces 37 which abut on an inner surface of the retracted cover sleeve. That means as long as the cover sleeve 20 is in its retracted position the release arms 32 remain deflected.

The user may interrupt the dispensing movement by pressing the button 4. The controller stops the motor and thus the plunger rod movement. The dispensing can be continued when the button 4 is pressed again.

The controller displays a holding time indicating a period during that the user has to hold the autoinjector 1 onto the injection site after the injection to ensure that the drug is completely administered and absorbed. The controller may display a down counter informing the user about the remaining holding time. The counter is implemented by a progression bar provided by a display visible from outside. Furthermore, the controller can drive the motor without moving the piston rod at idle speed to generate a motor sound which should prevent the user to remove the autoinjector from the injection site too early.

Once the holding time is elapsed a notification in form of a sound is emitted indicating the user that the autoinjector can be removed from the injection site. Alternatively, the end of the holding time may be indicated only by a LED.

When the user removes the autoinjector 1 from the injection site compressed cover sleeve spring 46 can release and thus it can move the cover sleeve 20 back distally into the needle covering position.

Upon movement of the cover sleeve 20 into its covering position the side support surfaces 37 get out of contact with the inner surface of the cover sleeve 20 and the release arms 32 can pivot radially outwards back into their locking position in which the free end of the arms 32 abut the contact surface inside the cover sleeve 20 thereby locking the cover sleeve 20 in its covering position. Hence, after the injection process the cover sleeve 20 cannot be moved into the retracted position again.

Upon completion of the injection the controller sends recorded injection data with a time stamp to an external receiver, e. g. a cloud server.

Furthermore, as the actuation arms 66 are not deflected anymore and thus do not contact the distal stop surface of the syringe holder 30 the entire syringe unit 2 can be pulled out of the drive unit by the user. The user can now discard the syringe unit 2 and the drive unit 3 is ready to be loaded with a new syringe unit.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF DESIGNATIONS

| | | | | |
|---|---|---|---|---|
| 1 | autoinjector | 50 | trigger sleeve | |
| 2 | syringe unit | 51 | trigger sleeve connector | |
| 3 | drive unit | | | |
| 4 | button | 52 | ledge | |
| | | 53 | trigger sleeve spring | |
| 10 | cap | 55 | counter sloped guiding surface | |
| 11 | holding tongue | | | |
| 12 | holder | | | |
| 13 | recess | 60 | gripper sleeve | |
| 15 | prefilled syringe | 61 | clamping arms | |
| 16 | piston | 62 | clamp | |
| 17 | rigid needle shield RNS | 64 | gripper sleeve spring | |
| 18 | injection needle | 65 | sloped guiding surface | |
| 19 | bearing surface | 66 | actuation arm | |
| | | 67 | free end | |
| 20 | cover sleeve | | | |
| 21 | first opening | 80 | support structure | |
| 22 | second opening | 83 | mechanics sleeve | |
| 23 | third opening | 87.1 | first shell | |
| 24 | medicament window | 87.2 | second shell | |
| 25 | label | 88 | bearing | |
| 26 | RFID tag | | | |
| 27 | rib | 92 | electronics | |
| 28 | protrusion | 94 | battery | |
| 29 | stop surface | 95 | LED | |
| | | 96.1 | first switch | |
| 30 | syringe holder | 96.2 | second switch | |
| 31 | end bulge | 97 | pin | |
| 32 | release arm | 98 | cam | |
| 33 | end ledge | | | |
| 34 | actuation surface | 102 | syringe unit | |
| 35 | center | 110 | cap | |
| 36 | nose | 114 | groove | |
| 37 | side support surface | 115 | straight portion | |
| 38.1 | distal portion | 116 | angled portion | |
| 38 | rocker | 120 | cover sleeve | |
| 38.2 | proximal portion | | | |
| 40 | plunger rod | | | |
| 41 | threaded rod | | | |
| 45 | cover sleeve connector | | | |
| 46 | cover sleeve spring | | | |
| 47 | syringe connector | | | |
| 48 | syringe connector spring | | | |
| 49 | flange | | | |

## Claims

1. A safety mechanism for an injection device (1) for dispensing a liquid drug from a reservoir (15) through a needle (18), the safety mechanism comprising an assembly (2) defining a longitudinal axis and wherein the assembly comprises
- a cap (10) releasably attachable to a distal end portion of the assembly (2), the cap (10) comprising a deflectable holding member (11) adapted to hold the cap (10) on the distal end portion;
- a rocker (38) tiltable around a center (35), the center dividing the rocker in a first portion (38.2) and a second portion (38.1)
**characterized in that** the first portion (38.2) includes an actuation surface (34) to tilt the rocker (38) when actuated and the second portion (38.1) is engageable to the holding member (11), wherein in a first tilt position of the rocker the cap (10) is held on the distal end by the holding member (11) and in a second tilt position the holding member (11) is deflected by the second portion (38.1) such that the cap (10) is released from the distal end portion.

2. Safety mechanism according to claim 1, wherein the rocker (38) is tiltable around an axis perpendicular to the longitudinal axis.

3. Safety mechanism according to claim 1 or 2, wherein the safety mechanism further comprises a reservoir holder (30) adapted to fixedly hold the reservoir (15), wherein the rocker (38) is formed integral with the reservoir holder (30) as a monolithic part.

4. Safety mechanism according to any of claims 1 to 3, wherein the safety mechanism further comprises a deflectable actuation arm (66) and a trigger member (50) movable relative to the rocker (38) and engageable to the actuation arm (66), wherein a relative axial movement between the trigger member (50) and the actuation arm (66) causes radial movement of the actuation arm (66) which causes the rocker (38) to move from the first tilt position to the second tilt position.

5. Safety mechanism according to claim 4, wherein the trigger member (50) is movable along the longitudinal axis and the actuation arm (66) is deflectable in a radial direction.

6. Safety mechanism according to claim 4 or 5, wherein the trigger member (50) comprises a first guiding surface (55) and the actuation arm (66) comprises a second guiding surface (65), wherein a movement of the trigger member (50) along the longitudinal axis causes the first guiding surface (55) to interact with the second guiding surface (65) thereby deflecting the actuation arm (66) in the radial direction.

7. Safety mechanism according to any of claims 4 to 6, further comprising a deflectable release arm (32) adapted to engage the actuation surface (34) of the rocker (38), wherein the release arm (32) is arranged inbetween the actuation arm (66) and the actuation surface (34), wherein a deflection of the actuation arm (66) causes a deflection of the release arm (32) thereby moving the rocker (38) from the first position to the second position.

8. Safety mechanism according to any of claim 1 to 7, wherein the assembly comprises a sensor arranged in a distal portion of the assembly and adapted to sense the cap (10), wherein the sensor is configured to provide a first sensor signal when the holding member (11) holds the cap and a second signal different from the first signal when the cap (10) is released from the holding member (11).

9. An injection device (1) for dispensing the liquid drug from the reservoir (15), the injection device (1) comprising, a plunger rod (40) and a drive for driving the plunger rod (40), wherein the injection device (1) further includes the safety mechanism according to any of claims 4 to 8.

10. Injection device (1) according to claim 9, comprising a reservoir unit adapted to hold the reservoir (15) and wherein the injection device (1) further comprises a drive unit (3) comprising the drive and wherein the reservoir unit (2) is releasably attachable to the drive unit (3).

11. Injection device (1) according to claim 10, wherein the drive unit (3) includes a movable clamping arm (61) that can be in an engaged position in which the arm (61) engages a protrusion (33) of the reservoir unit (2) to hold the reservoir unit in an attached state and wherein the clamping arm (61) can be in a released position in which the protrusion (33) is released and wherein the drive unit (3) includes a sensor configured to sense when the clamping arm (61) is in the engaged position.

12. Injection device (1) according to any of claims 9 to 11, wherein the drive is provided by a mechanical spring, which is biased manually by a user prior to an injection.

13. Injection device (1) according to any of claims 9 to 11, wherein the drive is provided by an electric motor and a controller adapted to control the motor.

14. Injection device (1) according to claim 13, wherein the motor is adapted to rotate a drive member (41) which is operatively coupled to the plunger rod (40) and the trigger member (50), wherein rotating the drive member (41) in a first rotational direction causes the plunger rod (40) and the trigger member (50) to move in a first direction along the longitudinal axis and rotating the drive member (41) in a second, counter rotational direction causes the plunger rod (40) and the trigger member (50) to move in a second direction along the longitudinal axis.

15. A method for operating a safety mechanism for an injection device (1) for dispensing a liquid drug from a reservoir (15) through a needle (18), the method includes the steps of
a. Providing an injection device defining a longitudinal axis;
b. Moving a trigger member (50) of the injection device along the longitudinal axis;
c. Tilting, by the trigger member movement, a rocker (38) from a first tilt position to a second tilt position;
d. Releasing, by the tilting movement of the rocker, a cap (10) releasably attached to a distal end portion of the injection device.
